# EUROPÄISCHE PATENTSCHRIFT

(11) **EP 1 280 534 B1**
(45) Veröffentlichungstag und Bekanntmachung des Hinweises auf die Patenterteilung: **16.07.2008**
(21) Anmeldenummer: 01925465.5
(22) Anmeldetag: 15.03.2001
(51) Int. Cl.: A61K 31/64, A61K 9/16, A61K 9/20

(54) **TORASEMID ENTHALTENDE LAGERSTABILE PHARMAZEUTISCHE ZUBEREITUNGEN**
TORASEMIDE-CONTAINING STORAGE STABLE PHARMACEUTICAL PREPARATIONS
PREPARATIONS PHARMACEUTIQUES STABLES AU STOCKAGE CONTENANT DU TORASEMIDE

(30) Priorität: 17.03.2000 DE 10013289
(43) Veröffentlichungstag der Anmeldung: 05.02.2003
(73) Patentinhaber: ABBOTT LABORATORIES, Abbott Park, Illinois 60064 (US)
(72) Erfinder: MAEGERLEIN, Markus, 68167 Mannheim (DE); HANTKE, Thomas, 68165 Mannheim (DE); BREITENBACH, Jörg, 68199 Mannheim (DE); ROSENBERG, Jörg, 67158 Ellerstadt (DE)
(74) Vertreter: Kinzebach, Werner
(86) Internationale Anmeldenummer: PCT/EP2001/002940
(87) Internationale Veröffentlichungsnummer: WO 2001/068100

(56) Entgegenhaltungen:
- EP-A- 0 852 140
- WO-A-01/10441
- WO-A-96/26197
- WO-A1-93/00097
- US-A- 5 914 336
- KNAUF H. ET AL: 'Clinical Pharmacokinetics and Pharmacodynamics of Torasemide' CLINICAL PHARMACOKINETICS Bd. 34, Nr. 1, Januar 1998, Seiten 1 - 24
- BURGER A.; WACHTER H.: 'Hunnius Pharmazeutisches Wörterbuch', 1993, WALTER DE GRUYTER, BERLIN * Seite 75 *
- ROLLINGER J.M. ET AL: 'Crystal forms of torasemide: new insights' EUROPEAN JOURNAL OF PHARMACEUTICS AND BIOPHARMACEUTICS Bd. 53, 2002, Seiten 75 - 86

## Beschreibung

Die vorliegende Erfindung betrifft Zubereitungen des Wirkstoffs Torasemid gemäß der Ansprüche 1-7, in denen das Torasemid im wesentlichen nicht-kristallin vorliegt. Weiterhin betrifft die Erfindung ein Verfahren zur Herstellung solcher Zubereitungen gemäß Anspruch 8 sowie Arzneiformen, enthaltend solche Zubereitungen gemäß Anspruch 9.

Torasemid (1-Isopropyl-3-[(4-m-toluidino-3-pyridyl)sulfonyl]-harnstoff ist ein Schleifendiuretikum, welches in unterschiedlichen Dosierungen zur Behandlung von Hypertonie, Ödemen und Niereninsuffizienz eingesetzt wird.

Von Torasemid sind bisher drei polymorphe Formen bekannt. Die Modifikationen I und II sind in Acta Cryst., 1978, S 2659-2662 und Acta Cryst., 1987, S. 1304-1310, beschrieben. Aus US Re 34,672 und 34,580 ist bekannt, dass sich die Modifikation II rasch in die Modifikation I umlagert.
Aus der US-A 5,914,336 ist eine weitere Modifikation III bekannt, die auch als Gemisch mit der Modifikation I eingesetzt werden kann.

In der WO 93/00097 sind lagerstabile Formulierungen des Torasemids beschrieben, in denen der Wirkstoff vorzugsweise in Form der Modifikation I eingesetzt wird.

Dieses Vorgehen birgt mehrere Nachteile. Der Wirkstoff muß nach der Synthese aufwendig und teuer weiterverarbeitet werden, um die benötigte Modifikation I zu erhalten. Weiterhin muß der Wirkstoff in einer eng definierten Teilchengröße vorliegen (90 % ≤ 96 µm und 50 % ≤ 48 µm), damit die gewünschte schnelle Wirkstofffreisetzung erreicht wird. Dies macht entsprechende Mahl- und Klassifizierungsschritte notwendig.

Die WO 01/10441 beschreibt polymorphe Formen von Torasemid einschließlich Torasemid Form V, und amorphes Torasemid.

Die EP-A 0852140 beschreibt ein Verfahren zur Herstellung einer festen Dispersion eines schwer wasserlöslichen Wirkstoffs, wobei man einen schwer wasserlöslichen Wirkstoff, ein den amorphen Zustand induzierendes Mittel und ein den amorphen Zustand stabilisierendes Mittel einer Wärmebehandlung oder mechanochemischen Behandlung unterwirft.

Aufgabe der vorliegenden Erfindung war es, Torasemid enthaltende pharmazeutische Zubereitungen bereitzustellen, die die mit dem Auftreten von polymorphen Formen einhergehende Nachteile vermeiden helfen.

Demgemäß wurden lagerstabile feste oder halbfeste Zubereitungen gefunden, die Torasemid in im Wesentlichen nicht-kristalliner Form, mindestens eine Bindemittelkomponente und gegebenenfalls weitere pharmazeutisch akzeptable Hilfsstoffe enthalten, wobei das Torasemid als homogen in einer Bindemittelmatrix dispergierte amorphe Agglomerate einer Größe von ≤ 1 µm oder als feste Lösung in einer Bindemittelmatrix vorliegt.

Im wesentlichen nicht-kristallin bedeutet im Sinne dieser Erfindung, dass nicht mehr als 5 %%, bevorzugt nicht mehr als 2 % %, des Wirkstoffs in Form von Kristallen vorliegt. Besonders bevorzugt sind Zubereitungen, die frei von kristallinem Wirkstoff sind.

Der Begriff der festen Lösung ist dem Fachmann bekannt und beschreibt im wesentlichen molekulardisperse Systeme, in denen der Wirkstoff homogen in einer als Lösungsmittel dienenden Bindemittelmatrix dispergiert ist.

Im Sinne dieser Erfindung bedeutet Torasemid auch die entsprechenden pharmakologisch akzeptablen Salze wie beispielsweise Salze mit organischen Säuren wie Essigsäure, Maleinsäure, Fumarsäure, Äpfelsäure, Weinsäure, Ascorbinsäure, Methansulfonsäure, Zitronensäure oder mit anorganischen Säure wie Salzsäure, Bromwasserstoffsäure, Phosphorsäure oder Schwefelsäure.

Erfindungsgemäß ist die Bindemittelmatrix in wässrigen Systemen zumindest teilweise löslich oder quellbar.

Als Bindemittelkomponenten kommen insbesondere thermoplastisch verarbeitbare Komponenten in Betracht.

Vorzugsweise enthalten die Zubereitungen mindestens eine Bindemittelkomponente ausgewählt unter:
Homo- und Copolymeren von N-Vinylverbindungen wie N-vinyllactamen, beispielsweise N-vinylcaprolactam oder N-vinylpiperidon, N-Vinylformamid oder N-Vinylimidazol; insbesondere Homo- und Copolymere des N-Vinylpyrrolidons (NVP) mit K-Werten nach Fikentscher im Bereich von 10 bis 100, bevorzugt 17 bis 90, besonders bevorzugt im Bereich von K 30 (Vgl. H. Fikentscher, Cellulosechemie 13 (1932), S. 58-64 und 71-74, wie Polyvinylpyrrolidon (PVP), Copolymere mit Vinylestern, insbesondere N-Vinylacetat, beispielsweise Copolymere aus 60 Gew.-% NVP und 40 Gew.-% Vinylacetat;
Acrylathaltige Polymeren wie Polyacrylaten, Polymethacrylaten, Copolymeren der Acrylsäure oder der Methacrylsäure, insbesondere deren Copolymeren mit Alkylestern der Acrylsäure oder Methacrylsäure wie Ethylacrylat, Butylacrylat oder Dialkylaminoalkylestern;
Solche Polymere sind beispielsweise unter der Handelsbezeichnung Eudragit^{®} kommerziell erhältlich.

Cellulosederivaten, insbesondere Cellulosestern und Celluloseethern wie Alkylcellulosen, beispielsweise Methylcellulose (M̅ᵣ 20000 bis 150000) oder Ethylcellulose, Hydroxyalkylcellulosen, beispielsweise Hydroxypropylcellulose (M̅ᵣ 60000 bis 1,2 Mio), Hydroxyalkyl-Alkylcellulosen, beispielsweise HydroxypropylMethylcellulose (M̅ᵣ 10000 bis 150000), Cellulosephthalate, beispielsweise Celluloseacetatphthalat (M̅ᵣ 40000);
Polyethylenglykole mit Molekulargewichten im Bereich von 400 bis 100000, bevorzugt 4000 bis 20000;
modifizierten Stärken oder Stärkeabbauprodukten wie beispielsweise Maltodextrin;
niedermolekulare Matrixkomponenten wie Zuckeralkoholen, beispielsweise Maltit, Mannit, Sorbit, Xylit, Erythrit oder Isomalt;
natürlichen oder überwiegend natürlichen Bindemitteln wie Gelatine, Xanthangummi, Alginaten, Polylactiden, Polyaminosäuren oder Mannanen, beispielsweise Galactomannan;

Es können auch Mischungen der genannten Polymere eingesetzt werden.

Besonders bevorzugte Bindemittel sind Homo- und Copolymere des N-Vinylpyrrolidons mit K-Werten von 17 bis 30, insbesondere ein Copolymer mit Vinylacetat der Zusammensetzung VP/VAc 60/40.

Die erfindungsgemäßen Zubereitungen können den Wirkstoff in Mengen von 0,5 bis 95 Gew.-%, vorzugsweise 5 bis 60 Gew.-%, besonders bevorzugt 5 bis 25 Gew.-%, enthalten.
Der Anteil an Hilfsstoffen kann dementsprechend 5 bis 99,5 Gew.-% betragen, wobei der Anteil an matrixbildenden Bindemitteln vorzugsweise 5 bis 99,5, besonders bevorzugt 40 bis 90 Gew.-% beträgt.

Weiterhin können die Zubereitungen noch 0 bis 94,5 Gew.-%, bevorzugt 5 bis 25 Gew.-%, übliche pharmakologisch akzeptable Hilfsstoffe enthalten, beispielsweise oberflächenaktive Substanzen wie Tenside, pH-beeinflussende Zusätze, Weichmacher, Füllstoffe, Gleitmittel, Stabilisatoren wie Konservierungsmittel oder Antioxidantien, Aromen, Farbstoffe oder geschmacksmaskierende Stoffe.

Als Tenside eignen sich beispielsweise Sucroseester, alkoxylierte Fettalkohole, alkoxylierte Fettsäuren oder Fettsäureglycerinester, ethoxylierte Sorbitanfettsäureester, polyoxyethyliertes hydriertes Rizinusöl, insbesondere

Weiterhin eignen sich Polyoxyethylen-polyoxypropylen-Blockcopolymere, die auch als Poloxamere bekannt sind, beispielsweise Poloxamer 407 oder Poloxamer 338.

Bevorzugt enthalten die erfindungsgemäßen Zubereitungen als Tenside ethoxyliertes hydriertes Rizinusöl, insbesondere PEG-35 oder PEG-40-, oder Poloxamere, insbesondere Poloxamer 407. Diese Tenside werden bevorzugt in Mengen von 5 bis 15 Gew.-% eingesetzt.

Als pH-beeinflussende Zusätze eigenen sich beispielsweise organische Carbonsäuren oder deren physiologisch akzeptable Salze - wie z.B. Essigsäure, Maleinsäure, Weinsäure, Zitronensäure -, Zuckersäuren wie z.B. Ascorbinsäure, Aminosäuren wie z.B. Glutaminsäure oder Argininsäure, weiterhin anorganische Säuren oder deren Salze wie beispielsweise Carbonate, Hydrogencarbonate, Phosphorsäure, Hydrogenphosphate oder Dihydrogenphosphate.

Als pH-beeinflussende Zusätze werden insbesondere Zitronensäure und Natriumacetat eingesetzt. Diese Zusätze können in Mengen von 0,1 bis 20 Gew.-%, vorzugsweise 2 bis 5 Gew.-%, bezogen auf die Gesamtmenge der Zubereitung, eingesetzt werden.

Die Herstellung der erfindungsgemäßen Zubereitungen kann durch Sprüheinbettung, Sprühtrocknung, Copräzipitation oder Lyophilisation erfolgen. So kann man beispielsweise den Wirkstoff gemeinsam mit den Matrixkomponenten in Wasser oder einem organischen Lösungsmittel wie beispielsweise Methanol, Ethanol, Isopropanol, Methylenchlorid, Toluol oder bevorzugt Tetrahydrofuran lösen und anschließend durch Einkomponentendüsen, Mehrkomponentendüsen oder über rotierende Scheiben versprühen.

Die Herstellung der erfindungsgemäßen Zubereitungen erfolgt vorzugsweise durch Schmelzeverfahren. Dazu wird zunächst eine homogene Schmelze der Einsatzstoffe hergestellt, die anschliessend extrudiert und der Formgebung unterworfen wird. Man kann eine Vormischung aller Komponenten aufschmelzen oder zunächst eine Schmelze der Hilfsstoffe herstellen und dann den Wirkstoff zudosieren.

Die Herstellung der Schmelze kann in an sich bekannten geeigneten Vorrichtungen wie beheizbaren Rührkesseln oder Knetern bei Schmelzetemperaturen von 40 bis 170°C, vorzugsweise ... bis 140°C, erfolgen. Die homogene Schmelze wird dann üblicherweise durch eine Düse oder eine Lochplatte extrudiert. Vorzugsweise erfolgt die Verarbeitung der Schmelze in einem Schneckenkneter oder Schneckenextruder, vorzugsweise in einem Doppelschneckenextruder. Die aus der Düse oder der Lochplatte austretenden noch thermoplastischen Stränge können beispielsweise durch übliche Abschlagtechniken wie Heiss- oder Kaltabschlag zu Granulaten geformt werden. Man kann die erstarrten Stränge auch durch geeignete Mahlverfahren zu Granulaten verarbeiten. Man kann die noch thermoplastischen Stränge auch nach dem beispielsweise aus der EP-A 240 906 bekannten Kalandrierverfahren direkt zu Tabletten verformen.

Das Verfahren wird vorzugsweise in Abwesenheit von Wasser oder organischen Lösungsmitteln durchgeführt. Es kann sich jedoch empfehlen bis zu 3 Gew.-% an Wasser als weichmachenden Zusatz einzusetzen. Dieses Wasser kann gewünschtenfalls auch vor der Extrusion der Schmelze durch Anlegen eines Vakuums entfernt werden.

Die Erfindung betrifft auch feste oder halbfeste, lagerstabile Arzneiformen, vorzugsweise feste Arzneiformen für die perorale Anwendung.

Die erfindungsgemässen Zubereitungen können als Tabletten, Filmtabletten, als Füllung für Hart- oder Weichgelatine-Kapseln oder Sachets, als Granulate oder Trinkgranulate eingesetzt werden.

Darüber hinaus können die erfindungsgemässen Zubereitungen auch in nicht-peroralen Arzneiformen, wie zum Beispiel Suppositorien, eingesetzt werden.

So kann gemahlenes Extrudat mit für die Tablettierung üblichen Hilfsstoffen wie Bindemitteln, Füllmitteln, Sprengmitteln, Fliessregulierungsmitteln oder Formertrennmitteln gemischt und anschliessend auf einer konventionellen Tablettenpresse zu Tabletten gepresst werden. Die Korngrösse des gemahlenen Extrudats beträgt vorzugsweise < 1500 µm. Die Tablettiermischung kann auch ein Matrixretardierungsmittel enthalten.

Die Tabletten können auch mit einem Filmüberzug versehen werden. Auf diesem Wege können auch magensaftresistente Filmtabletten hergestellt werden, oder Filmtabletten mit einem die Wirkstoff-Freisetzung verzögernden Überzug, beispielsweise eine retardierende Polymere vom Eudragit-Typ enthaltenden Überzug.

Die erfindungsgemässen Zubereitungen können auch als Pulvermischung mit üblichen Hilfsstoffen in Hartgelatinekapseln oder in Sachets gefüllt werden oder als Füllung für Weichgelatine-Kapseln dienen.

Die erfindungsgemässen Zubereitungen werden dabei in solchen Mengen eingesetzt, dass pro Dosiseinheit typischerweise 2,5 bis 200 mg, bevorzugt 2,5 bis 20 mg, Torasemid enthalten sind.

Man kann auch Kombinationsarzneiformen mit weiteren Diuretika herstellen, beispielsweise mit Furosemid, Hydrochlorthiazid, Amilorid, Triamteren und Spironolacton.

Mit Hilfe der erfindungsgemässen Zubereitungen lassen sich lagerstabile Arzneiformen herstellen, in denen das Torasemid nicht-kristallin und vorzugsweise als feste Lösung oder amorph vorliegt. Mit Hilfe von DSC-Messungen (Differential Scanning Calorimetry)) oder WAXS-Aufnahmen (WideAngle X-Ray-Spectroscopy-Weitwinkelröntgenstreung) lässt sich zeigen, daß die Zubereitungen keine kristallinen Anteile aufweisen. Auf diese Weise kann das Problem der unterschiedlichen Stabilität und Bioverfügbarkeit der polymorphen Formen vermieden werden.

### Beispiele

### Beispiele 1 bis 6

In einem Einschneckenextruder (Schneckenlänge 170 mm, Schneckendurchmesser 6,4 mm, wurden die in Tabelle I aufgeführten Mischungen aufgeschmolzen und extrudiert. Die drei Heizzonen des Extruders wiesen folgendes Temperaturprofil auf: Zone 1: 65 bis 76°C, Zone 2: 100 bis 130°C, Zone 3: 110 bis 140°C. Die Drehzahl der Schnecke betrug 120 bis 180 U/min.

Die aus der Düse austretenden homogenen Stränge wurden im erstarrten Zustand zu Granulaten mit einer Korngröße < 1500 µm gemahlen.

**Tabelle I: (Angaben in Gew.-%)**

| Formulierung Nr. | 1 | 2 | 3 | 4 | 5 | 6 |
|---|---|---|---|---|---|---|
| Torasemid | 20 | 20 | 20 | 20 | 20 | 20 |
| Kollidon VA 64 | 75 | 75 | 75 | 70 | 70 | |
| Kollidon K17 | | | | | | 75 |
| Zitronensäure anh. | | | | 5 | | |
| Cremophor® RH 40 | 5 | | | | | |
| Natriumacetat anh. | | | | | 5 | |
| Poloxamer 407 | | 5 | | 5 | 5 | 5 |
| Saccharosemonopalmitat | | | 5 | | | |

| | | | | | | |
|---|---|---|---|---|---|---|
| Kollidon® VA 64: Copolymer aus 60 Gew.-% N-Vinylpyrrolidon und 40 Gew.-% Vinylacetat, Firma BASF Kollidon K17: Polyvinylpyrrollidon, K-Wert 17, Firma BASF Cremophor RH 40: PEG-40-hydrogeniertes Rizinusöl, Firma BASF | | | | | | |

### Beispiel 7: Herstellung von Tabletten

Gemahlenes Extrudat (wie in den Formulierungen Nr. 1 bis 6 beschrieben) wird mit den unten angegebenen Hilfsstoffen in den entsprechenden Mengen gemischt und zu gewölbten Tabletten mit einem Gewicht von 200 mg und einem Durchmesser von 8 mm gepresst.

| | |
|---|---|
| Formulierung 1 | 50 mg |
| Crosscarmelose | 10 mg |
| CaHPO₄ anhydr. | 136 mg |
| Aerosil 200⁺⁾ | 2 mg |
| Magnesiumstearat | 2 mg |

| | |
|---|---|
| +): hochreines Silicumdioxid; BET-Oberfläche 200 ±25 m², mittl. Grösse der Primärteilchen 12 nm | |

### Beispiel 8: Herstellung von matrixretardierten Tabletten

Analog Beispiel 7 werden Tabletten der folgenden Zusammensetzung hergestellt:

| | |
|---|---|
| Formulierung 1 | 50 mg |
| CaHPO₄ | 133 mg |
| Crosscarmelose | 5 mg |
| Eudragit RL⁺⁾ | 8 mg |
| Aerosil 200 | 2 mg |
| Magnesiumstearat | 2 mg |

| | |
|---|---|
| +): Eudragit RL: Poly(ethylacrylat, methylmethacrylat, trimethylammonium-methacrylatchlorid (1:2:0,2), Fa. Roehm | |

### Beispiel 9: Herstellung von Filmtabletten mit modifizierter Wirkstofffreisetzung

Die Tabletten gemäß Beispiel 7 werden mittels eines konventionellen Sprühverfahrens in einem Coater mit einem Filmüberzug versehen.

| | |
|---|---|
| Tablettengewicht | 204,55 mg |
| Zusammensetzung des Überzugs: | |
| Eudragit L100-55 2,28 mg | |
| Polyoxyethylen-20-sorbitammonooleat | 0,04 mg |
| NaOH | 0,03 mg |
| Simethicone | 0,01 mg |
| Talkum | 1,52 mg |
| Macrogol 6000 | 0,67 mg |

### Beispiel 10: Hartgelatinekapseln; Sachets

Hartgelatinekapseln der Grösse 1 oder Sachets werden mit 100 mg der homogenen Pulvermischung der folgenden Zusammensetzung befüllt:

| | |
|---|---|
| Formulierung 1 | 40 mg |
| Mannitol | 49,5 mg |
| Aerosil 200 | 0,5 mg |

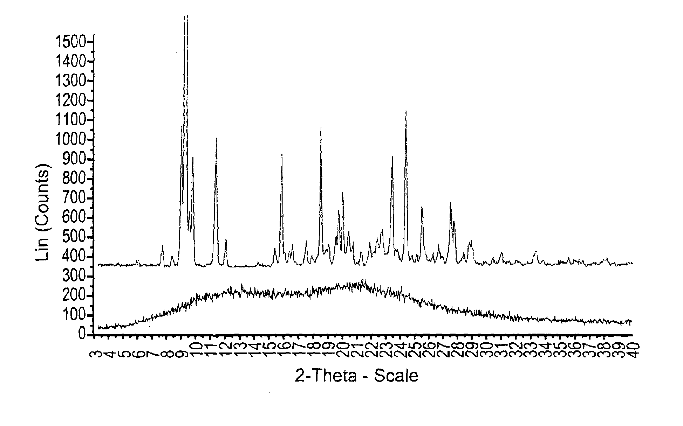

In der Abbildung sind die WAXS-Aufnahmen von Torasemid als kristalline Rohware (obere Linie) und von Torasemid-Extrudat (untere Linie) abgebildet.

## Patentansprüche

1. Lagerstabile feste oder halbfeste pharmazeutische Zubereitung, enthaltend Torasemid in im Wesentlichen nicht-kristalliner Form, mindestens eine Bindemittelkomponente und gegebenenfalls weitere pharmazeutisch akzeptable Hilfsstoffe, wobei das Torasemid als homogen in einer Bindemittelmatrix dispergierte amorphe Agglomerate einer Größe von ≤ 1 µm oder als feste Lösung in einer Bindemittelmatrix vorliegt.

2. Zubereitungen nach Anspruch 1, enthaltend Torasemid in Form einer festen Lösung in einer Bindemittelmatrix.

3. Zubereitungen nach Anspruch 1, in denen das Torasemid als homogen in einer Bindemittelmatrix dispergierte amorphe Agglomerate einer Größe von ≤ 1 µm vorliegt.

4. Zubereitungen nach einem der Ansprüche 1 bis 3, enthaltend mindestens eine Bindemittelkomponente ausgewählt aus der Gruppe bestehend aus Homo- und Copolymeren des N-Vinylpyrrolidons.

5. Zubereitungen nach einem der Ansprüche 1 bis 4, enthaltend
a) 0,5 bis 95 Gew.-% Torasemid
b) 5 bis 99,5 Gew.-% mindestens einer Bindemittelkomponente
c) 0 bis 94,5 Gew.% weitere pharmazeutisch akzeptable Hilfsstoffe, wobei die Summe der Komponenten a), b) und gegebenenfalls c) 100 Gew.% beträgt.

6. Zubereitungen nach einem der Ansprüche 1 bis 5, enthaltend 0,1 bis 20 Gew.-% eines Tensids.

7. Zubereitungen nach einem der Ansprüche 1 bis 6, enthaltend 0,1 bis 20 Gew.-% einer pH-stabilisierenden Verbindung.

8. Verfahren zur Herstellung einer pharmazeutischen Zubereitung gemäß einem der Ansprüche 1 bis 7, **dadurch gekennzeichnet, dass** man Torasemid mit mindestens einer Bindemittelkomponente und gegebenenfalls weiteren pharmazeutisch akzeptablen Hilfsstoffen in der Schmelze homogen vermischt und anschließend extrudiert.

9. Lagerstabile Arzneiformen für die perorale Verabreichung, enthaltend Zubereitungen gemäß einem der Ansprüche 1 bis 7.

## Claims

1. A storage-stable solid or semisolid pharmaceutical preparation, comprising torasemide in essentially noncrystalline form, at least one binder component and, if appropriate, further pharmaceutically acceptable excipients, the torasemide being present as amorphous agglomerates which are dispersed homogeneously in a binder matrix and have a size of ≤ 1 µm or as a solid solution in a binder matrix.

2. The preparation according to claim 1, comprising torasemide in the form of a solid solution in a binder matrix.

3. The preparation according to claim 1, in which the torasemide is present as amorphous agglomerates which are dispersed homogeneously in a binder matrix and have a size of ≤ 1 µm.

4. The preparation according to one of claims 1 to 3, comprising at least one binder component selected from the group consisting of homo- and copolymers of N-vinylpyrrolidone.

5. The preparation according to one of claims 1 to 4, comprising
a) 0.5 to 95% by weight of torasemide
b) 5 to 99.5% by weight of at least one binder component
c) 0 to 94.5% by weight of further pharmaceutically acceptable excipients, the sum of the components a), b) and, if appropriate, c) being 100% by weight.

6. The preparation according to one of claims 1 to 5, comprising 0.1 to 20% by weight of a surfactant.

7. The preparation according to one of claims 1 to 6, comprising 0.1 to 20% by weight of a pH-stabilizing compound.

8. A process for the production of a pharmaceutical preparation according to one of claims 1 to 7, which comprises homogeneously mixing torasemide with at least one binder component and, if appropriate, further pharmaceutically acceptable excipients in the melt and subsequently extruding.

9. A storage-stable pharmaceutical form for peroral administration, comprising a preparation according to one of claims 1 to 7.

## Revendications

1. Préparation pharmaceutique solide ou semi-solide, stable au stockage, contenant du torasémide sous forme essentiellement non cristalline, au moins un composant liant et le cas échéant, d'autres auxiliaires pharmaceutiquement acceptables, où le torasémide se présente sous la forme d'un agglomérat homogène amorphe, dispersé dans une matrice de liant, ayant une taille ≤ 1 µm ou sous forme d'une solution solide dans une matrice de liant.

2. Préparations selon la revendication 1, contenant le torasémide sous forme d'une solution solide dans une matrice de liant.

3. Préparations selon la revendication 1, dans lesquelles le torasémide se présente sous la forme d'un agglomérat homogène amorphe, dispersé dans une matrice de liant, ayant une taille ≤ 1 µm.

4. Préparations selon l'une des revendications 1 à 3, contenant au moins un composant liant choisi parmi le groupe consistant en des homo- et copolymères de la N-vinylpyrrolidone.

5. Préparations selon l'une des revendications 1 à 4, contenant
a) 0,5 à 95% en poids de torasémide,
b) 5 à 99,5% en poids d'au moins un composant liant,
c) 0 à 94,5% en poids d'autres auxiliaires pharmaceutiquement acceptables, où la somme des composants a), b) et le cas échéant c) se situe à 100% en poids.

6. Préparations selon l'une des revendications 1 à 5, contenant 0,1 à 20% en poids d'un tensioactif.

7. Préparations selon l'une des revendications 1 à 6, contenant 0,1 à 20% en poids d'un composé stabilisant le pH.

8. Procédé de préparation d'une préparation pharmaceutique selon l'une des revendications 1 à 7, **caractérisé en ce que** l'on mélange de manière homogène, le torasémide avec au moins un composant liant et le cas échéant, d'autres auxiliaires pharmaceutiques acceptables, dans la masse fondue, et ensuite, on extrude.

9. Forma galénique stable pour administration perorale, contenant les préparations selon l'une des revendications 1 à 7.
